# EUROPEAN PATENT APPLICATION

(11) **EP 1 219 611 A1**
(43) Date of publication of application: **03.07.2002**
(21) Application number: 00956884.1
(22) Date of filing: 01.09.2000
(51) Int. Cl.: C07D 267/18, C07D 413/06, C07D 207/16

(54) **NOVEL PROCESSES FOR PREPARING OXAZEPINE DERIVATIVES**

(30) Priority: 03.09.1999 JP 25029899
(71) Applicant: Ajinomoto Co., Inc., Tokyo 104-0031 (JP)
(72) Inventor: SEKIYAMA, Takaaki Ajinomoto Co., Inc., Kawasaki-shi Kanagawa 210-0801 (JP); MATSUZAWA, Toshihiro Ajinomoto Co., Inc., Yokkaichi-shi Mie 510-0885 (JP); YAMAMOTO, Takashi Ajinomoto Co., Inc., Kawasaki-shi Kanagawa 210-0801 (JP); YATAGAI, Masanobu Ajinomoto Co., Inc., Kawasaki-shi Kanagawa 210-0801 (JP); EZAKI, Junko Ajinomoto Co., Inc., Kawasaki-shi Kanagawa 210-0801 (JP)
(74) Representative: Nicholls, Kathryn Margaret
(86) International application number: JP0005967
(87) International publication number: WO0117980

(57) **Abstract**

A [2-(2-bromobenzyloxy)phenyl] amide derivative having a substituent introduced through amido bond, the substituent locating at the 5-position of the final compound, as the starting material, is subjected to the intramolecular arylation and then the obtained product is reduced. In particular, (R)-1-[(4-methoxyphenyl)acetyl]pyrrolidine-2-carboxylic acid [2-(2-bromo benzyloxy)phenyl]amide is subjected to the intramolecular arylation to obtain (R)-[[2-(5,11-dihydrodibenzo[b,e][1,4]oxazepine-5-carbonyl) pyrrolidin]-1-yl]-2-(4-methoxyphenyl)ethanone and then this product is reduced. This process is particularly useful for producing 5-substituted-5,11-dihydrodibenzo[b,e][1,4]oxazepine derivatives, particularly 5,11-dihydro-5-[1-(4-methoxyphenethyl)-2-pyrrolidinylmethyl]dibenzo- [b,e][1,4]oxazepine, on an industrial scale.

## Description

### Background of the Invention

The present invention relates to a process for producing 5-substituted-5,11-dihydrodibnenzo[b,e][1,4]oxazepine derivatives antagonistic to calcium channels and useful for treating or preventing abnormal motor functions of gastrointestinal tracts, particularly intestinal diseases such as irritable bowel syndrome. In particular, the present invention relates to a process for producing 5,11-dihydro-5-[1-(4-methoxyphenethyl)-2-pyrrolidinylmethyl]dibenzo[b,e][1,4]oxazepine.

Some of 5-substituted-5,11-dihydrodibenzo[b,e][1,4]oxazepine derivatives are known to be antagonistic to calcium channels and useful for treating or preventing abnormal motor functions of gastrointestinal tracts, particularly intestinal diseases such as irritable bowel syndrome (WO 97/33885, WO 99/12925 and WO 00/40570). For example, WO 97/33885 discloses (R)-(+)-5,11-dihydro-5-[1-(4-methoxyphenethyl)-2-pyrrolidinylmethyl]dibenzo[b,e][1,4]oxazepine of the following formula (6):

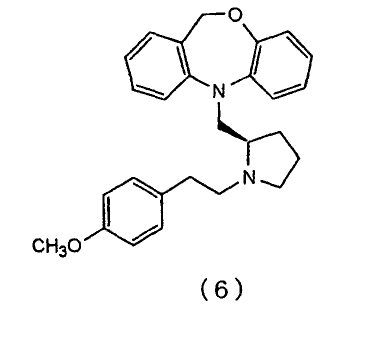

Although the following process for synthesizing (R)-(+)-5,11-dihydro-5-[1-(4-methoxyphenethyl)-2-pyrrolidinylmethyl]dibenzo[b,e]-[1,4]oxazepine (6) is shown in this Official Gazette, this process has a problem that by the reaction of starting 5, 11-dihydrodibenzo[b,e][1,4]oxazepine (7) with (S)-(+)-3-chloro-1-(4-methoxyphenethyl)piperidine (8), a large amount of (S)-5,11-dihydro-5-[1-[2-(4-methoxyphenyl)ethyl]piperidin-3-yl]dibenzo[b,e][1,4]oxazepine (9) is produced as a by-product in addition to the intended compound of the formula (6) and, therefore, a complicated industrial purification method such as column chromatography is necessitated:

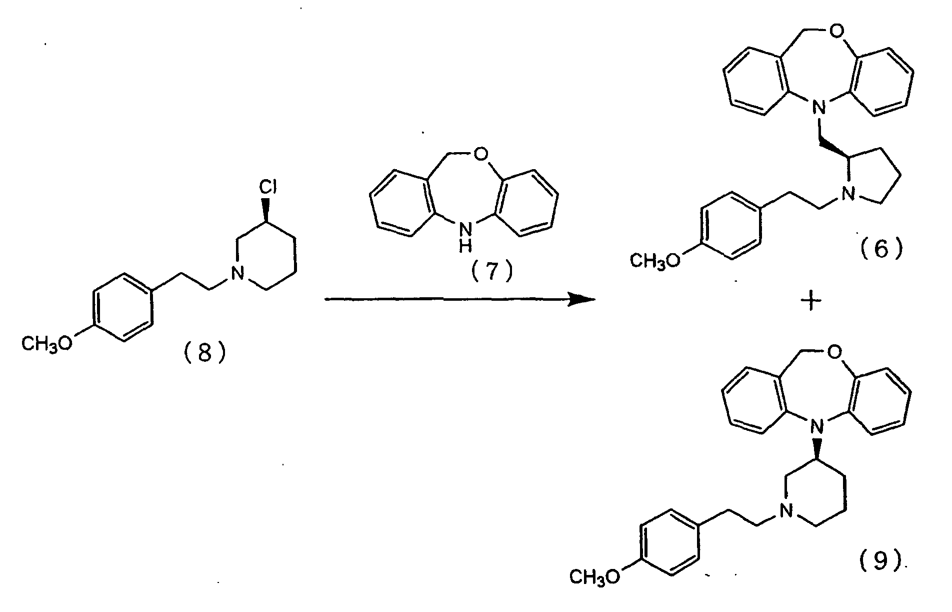

Further, EP 404359 discloses a process for producing a compound which is the same as that of the formula (6) except that the oxazepine part is replaced with thiazepine. Referring to the method described in EP 404359, the inventors tried to obtain the intended compound (6) by reducing a compound of the following formula (4) to produce (R)-2-(2-bromobenzyloxy)-N-[[1-[2-(4-methoxyphenyl)ethyl]pyrrolidin-2-yl]methyl]aniline (10) and then converting this product by the intramolecular arylation. However, a large amount of (R)-2-[N-[[1-[2-(4-methoxyphenyl)ethyl]pyrrolidin-2-yl]methyl]amino]phenol (11) was produced as a by-product in the reduction step to make the efficient production of the intended compound impossible.

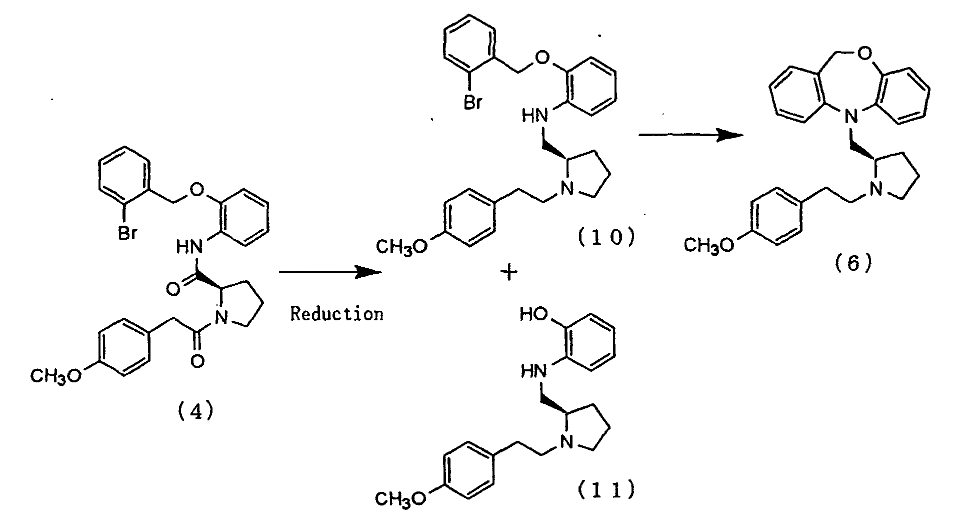

### Disclosure of the Invention

The object of the present invention is to establish an industrially useful process for producing 5-substituted-5,11-dihydrodibenzo [b,e][1,4]oxazepine derivatives, particularly 5,11-dihydro-5-[1-(4-methoxyphenethyl)-2-pyrrolidinylmethyl]dibenzo[b,e][1,4]oxazepine.

Another object of the present invention is to provide useful intermediates for producing 5,11-dihydro-5-[1-(4-methoxyphenethyl)-2-pyrrolidinylmethyl]dibenzo[b,e][1,4]oxazepine.

After intensive investigations made for the purpose of solving the above-described problems, the inventors have found that the intended 5-substituted-5,11-dihydrodibenzo[b,e][1,4]oxazepine derivatives (3) can be efficiently obtained by incorporating a substituent, which will be the substituent in the 5-position, into the molecule through an amido bond to obtain a compound of formula (1), then converting this compound into a compound of formula (2) by the intramolecular arylation, and finally reducing the obtained compound. The present invention has been completed on the basis of this finding.

Namely, the present invention provides a process for producing 5-substituted-5,11-dihydrodibenzo[b,e][1,4]oxazepine derivatives represented by the following formula (3) and stereoisomers thereof: 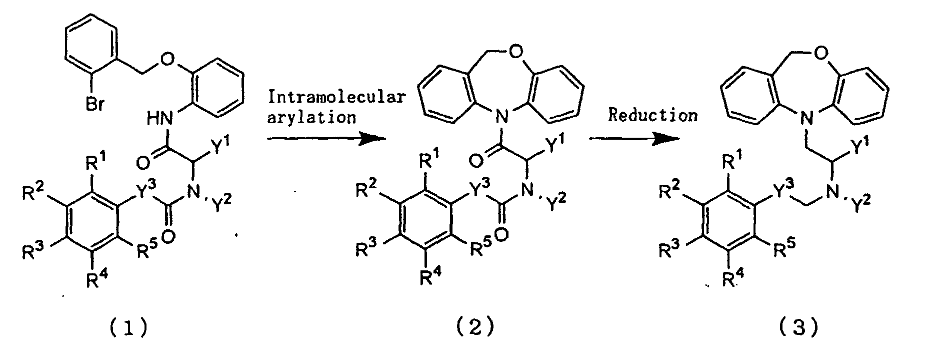 wherein Y¹ represents hydrogen atom, Y² represents hydrogen atom or a lower alkyl group, or Y¹ and Y² together represent CH₂-CH₂-CH₂ or CH₂-CH₂-CH₂-CH₂, Y³ represents CH₂ or CH₂-CH₂, and R¹ to R⁵ may be the same or different from one another and they each represent hydrogen atom, a halogen atom, a lower alkyl group, hydroxyl group, a lower alkoxyl group, amino group or a lower alkylamino group, or R¹ and R², R² and R³, R³ and R⁴ or R⁴ and R⁵ together form -OCH₂O-which comprises the steps of intramolecular-arylating a [2-(2-bromobenzyloxy)phenyl]amide derivative of the above-mentioned formula (1) to form a 5,11-dihydro-dibenzo[b,e][1,4]oxazepine derivative of the above-mentioned formula (2), and reducing this compound.

The present invention also provides a process for producing (R)-(+)-5,11-dihydro-5-[1-(4-methoxyphenethyl)-2-pyrrolidinylmethyl]-dibenzo[b,e][1,4]oxazepine represented by the following formula (6), which comprises the steps of intramolecular-arylating (R)-1-[(4-methoxyphenyl)acetyl]pyrrolidine-2-carboxylic acid [2-(2-bromo-benzyloxy)phenyl]amide of the following formula (4) to form (R)-[[2-(5,11-dihydro-dibenzo[b,e][1,4]oxazepine-5-carbonyl)pyrrolidin]-1-yl]-2-(4-methoxyphenyl)ethanone of the following formula (5), and reducing this compound:

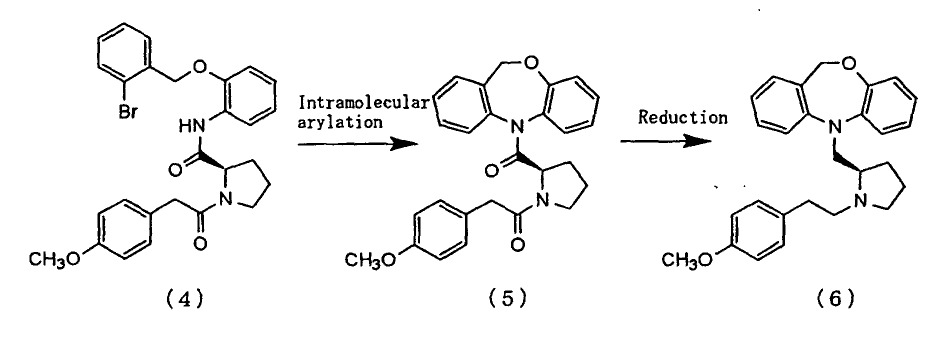

The present invention also provides a process wherein, in the process for producing the compound of above formula (6), (R)-[[2-(5,11-dihydro-dibenzo[b,e][1,4]oxazepine-5-carbonyl)pyrrolidin]-1-yl]-2-(4-methoxyphenyl)ethanone of the above formula (5) is crystallized and then the crystals are isolated.

The present invention further provides (R)-1-[(4-methoxyphenyl)acetyl]pyrrolidine-2-carboxylic acid [2-(2-bromo-benzyloxy)phenyl]amide of the above formula (4) which is an important starting material for the compound of the above formula (6), (R)-[[2-(5,11-dihydro-dibenzo[b,e][1,4]oxazepine-5-carbonyl)pyrrolidin]-1-yl]-2-(4-methoxyphenyl)ethanone of the above formula (5) which is an important intermediate, and crystals thereof.

### Best Mode for Carrying Out the Invention

The lower alkyl groups and also the lower alkyl groups in the lower alkylamino groups in the above formulae are linear or branched lower alkyl groups having 1 to 8 carbon atoms, preferably 1 to 4 carbon atoms. The lower alkoxyl groups are linear or branched lower alkoxyl groups having 1 to 8 carbon atoms, preferably 1 to 4 carbon atoms.

Preferably Y¹ and Y² in the above formulae together form CH₂-CH₂-CH₂. Y³ is preferably CH₂. R¹ to R⁵ may be the same or different from one another and preferably they each represent hydrogen atom or a lower alkoxyl group. It is particularly preferred that R¹, R², R⁴ and R⁵ each represent hydrogen atom and R³ represents a lower alkoxyl group (particularly methoxyl group).

The 2-(2-bromobenzyloxy)phenyl]amide derivatives of the above formula (1) used as the starting materials in the process of the present invention are produced by condensing an N-acylamino acid derivative (12) with 2-(2-bromobenzyloxy)aniline (13) or a salt thereof to form an amido bond. In the above formulae (1), (2) and (3), the term "lower alkyl groups" indicates alkyl groups having 1 to 8 carbon atoms, the term "lower alkoxyl groups" indicates alkoxyl groups having 1 to 8 carbon atoms, and the term "lower alkylamino groups" indicates alkylamino groups having 1 to 8 carbon atoms. The halogen atoms represent fluorine atom, chlorine atom, bromine atom, etc. For the condensation, a method usually employed for the condensation reaction such as acid anhydride mixed method, active ester method or DCC method can be employed.

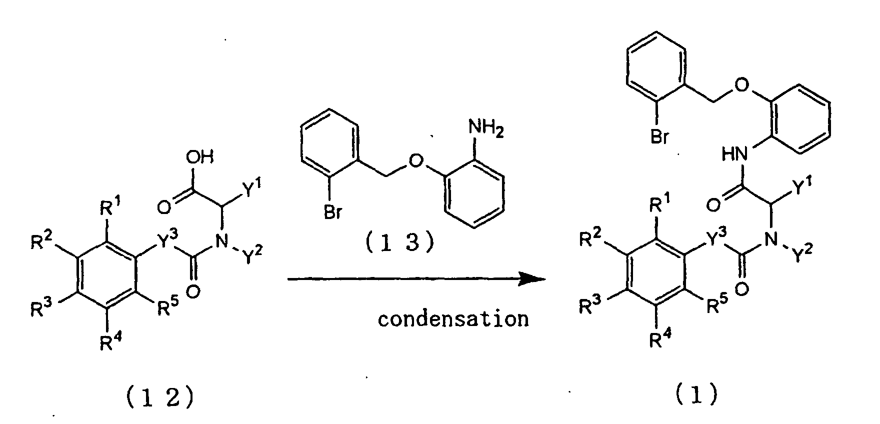

(R)-1-[(4-methoxyphenyl)acetyl]pyrrolidine-2-carboxylic acid [2-(2-bromobenzyloxy)phenyl]amide of the above formula (4) which is one of the compounds of the formula (1) in the present invention can be obtained by reacting D-proline (14) with 4-methoxyphenylacetyl chloride (15) to obtain (R)-1-[(4-methoxyphenyl)acetyl]pyrrolidine-2-carboxylic acid (16), converting this compound into an acid anhydride mixture by an ordinary method, and further reacting this product with 2-(2-bromobenzyloxy)aniline hydrochloride (17) as shown by the following reaction scheme. Although (R)-1-[(4-methoxyphenyl)acetyl]pyrrolidine-2-carboxylic acid [2-(2-bromobenzyloxy)phenyl]amide (4) obtained by the reaction can be directly used in the subsequent step, it is preferred to use this compound after purification by, for example, crystallization.

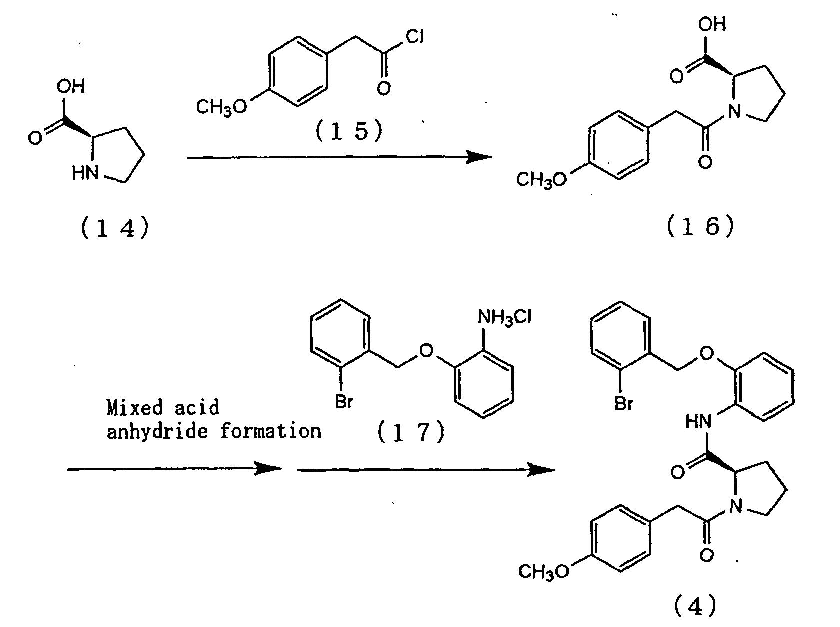

The intended 5-substituted-5,11-dihydro-dibenzo[b,e][1,4] oxazepine derivatives of the above formula (3) can be obtained by the intramolecular arylation of the 2-(2-bromobenzyloxy)phenyl]amide derivatives (1) obtained as described above to obtain 5,11-dihydro-dibenzo[b,e][1,4]oxazepine derivatives of the above formula (2) followed by the reduction of the obtained derivatives.

In the intramolecular arylation, the [2-(2-bromo-benzyloxy)phenyl]amide derivative (1) is dissolved in a solvent, then a metal catalyst such as copper or its salt and a suitable inorganic base are added to the obtained solution and the reaction is carried out in an inert gas stream at a temperature of 100 to 150°C which varies depending on the boiling point of the solvent for 8 to 200 hours. The solvents include toluene, pyridine, picoline, ethylpyridine, DMF, diphenyl ether, etc. The inorganic bases include sodium carbonate, potassium carbonate, etc. Preferably, the intramolecular arylation reaction is carried out by using cuprous bromide as the catalyst, 1 to 3 equivalents of potassium carbonate and pyridine or picoline as the solvent in an inert gas stream at 115 to 140°C for 10 to 100 hours.

The reaction mixture containing 5,11-dihydro-dibenzo [b,e][1,4]oxazepine derivative (2) obtained by the above-described reaction can be subjected to the subsequent reaction after the purification of the product by the extraction, silica gel chromatography or the like or, on the other hand, the reaction mixture can be directly used for the subsequent reaction. The purity of the intended final compound can be improved by crystallizing (R)-[[2-(5,11-dihydro-dibenzo[b,e][1,4] oxazepine-5-carbonyl)pyrrolidin]-1-yl]-2-(4-methoxyphenyl) ethanone (5) which is one of the compounds of the formula (2) of the present invention. The crystallization is conducted by, for example, dissolving a residue, containing the compound of the formula (5) obtained by the extraction from the reaction mixture, in a solvent such as toluene and then cooling the obtained solution to form the crystals. In this step, the compound of the formula (5) is dissolved in the solvent to obtain a solution having a concentration of 40 to 50 % by weight and then the crystallization is conducted at 20 to 30°C to obtain the crystals (crystals 1). When the compound of the formula (5) is dissolved in toluene to obtain a solution having a concentration of 10 to 30 % by weight and then the obtained solution is cooled to 10°C, other crystals can be obtained (crystals 2). The crystals 1 are formed in the form of glassy, flaky crystals on the walls of the vessel, while the crystals 2 are formed in the form of a suspension of fine particles. The crystals 1 can be converted into the crystals 2 by suspending crystals 1 in toluene and stirring the obtained suspension at 10 to 50°C.

The subsequent reduction reaction can be conducted by dissolving the 5,11-dihydro-dibenzo[b,e][1,4]oxazepine derivative (2) obtained as described above in a solvent and adding sodium borohydride and boron trifluoride / tetrahydrofuran complex to the obtained solution. As for the reaction conditions, the reaction is to be conducted in an inert gas stream at 5 to 60°C for 4 to 70 hours. The solvents are ethers such as tetrahydrofuran. The solvent may contain 0 to 50 % of toluene. Preferably, a suspension of 3 to 6 equivalents of sodium borohydride in tetrahydrofuran is cooled to 0 to 10°C and then a solution of a compound of the formula (2) in tetrahydrofuran is added to the suspension. 4 to 6 equivalents of boron trifluoride / tetrahydrofuran complex is added dropwise to the obtained solution, and they are stirred at 0 to 10°C for 1 to 20 hours and then at 30 to 40°C for 10 to 60 hours. The intended 5-substituted-5,11-dihydro-dibenzo[b,e][1,4]oxazepine derivative (3) can be obtained by adding 6 to 8 equivalents of an aqueous sodium hydroxide solution to the reaction mixture, stirring the obtained mixture at 50 to 60°C for 1 to 4 hours, extracting the product from the reaction mixture and purifying it by silica gel column chromatography or the like. The compound of the formula (3) extracted from the reaction mixture can be obtained in the form of crystals of a salt thereof with a suitable acid. The salt is, for example, hydrochloride.

### Examples

The conditions of the liquid chromatography in the analysis were as follows:

| | |
|---|---|
| Column | YMC-Pack ODS-AM AM-302 |
| | 4.6 mm I. D. x 150 mm |
| Solvent | A: 0.1 % trifluoroacetic acid |
| | B: acetonitrile |
| | A:B = 60:40→8:82 / 35 min |
| Flow rate | 1 mL/min |
| Detection method | UV 254 nm |
| Column temperature | 40°C |
| Injection volume | 10 µL |

### [Referential Example 1]

### Preparation of (R)-1-[(4-methoxyphenyl)acetyl]pyrrolidine-2-carboxylic acid (16):

D-proline (430 g, 3.73 mol) was dissolved in water (4.30 L). Acetone (2.15 L) and 6 M aqueous sodium hydroxide solution (0.645 L) were added to the obtained solution. The solution was cooled to 5°C and then a solution of 4-methoxyphenylacetyl chloride (696 g, 3.73 mol) in acetone (1.29 L) was added dropwise thereto. In this step, 6 M aqueous sodium hydroxide solution was also added dropwise thereto to keep pH in the range of 13 to 14. After the completion of the addition, the reaction mixture was concentrated to a volume of about 5.5 L under reduced pressure. The obtained concentrate was added dropwise to 6 M hydrochloric acid (1.29 L), and the resultant mixture was stirred overnight. The crystals thus obtained were filtered to obtain (R)-1-[(4-methoxyphenyl) acetyl]pyrrolidine-2-carboxylic acid (953 g, 95 %).
¹H NMR (CDCl₃) δ 1.93-2.11(m,3H), 2.32(m,1H), 3.48(m,1H), 3.59(m,1H), 3.67(s,2H), 3.78(s,3H), 4.58(m,1H), 6.86(d-like,2H), 7.18(d-like,2H), 9.90(br,1H).
¹³C NMR (CDCl₃) δ 24.8, 27.5, 40.8, 47.9, 55.3, 60.0, 114.2, 125.3, 130.0, 158.7, 172.6, 173.2.
ESI MASS m/z (MH⁺) 264.

### [Example 1]

### Preparation of (R)- 1-[(4-methoxyphenyl)acetyl]pyrrolidine-2-carboxyic acid [2-(2-bromobenzyloxy)phenyl]amide (4):

(R)-1-[(4-methoxyphenyl)acetyl]pyrrolidine-2-carboxyic acid (953 g, 3.55 mol) obtained in Referential Example 1 was suspended in ethyl acetate (12.1 L). The obtained suspension was cooled to 5°C and then N-methylmorpholine (441 mL, 4.01 mol) was added to the suspension. Ethyl chloroformate (373 mL, 3.90 mol) was added dropwise to the reaction mixture while it was kept at a temperature of not higher than 10°C. After the completion of the addition, the reaction mixture was stirred at 5°C for 1 hour. 2-(2-Bromobenzyloxy)aniline hydrochloride (1.19 kg, 3.55 mmol) was added to the obtained solution, and the reaction mixture was stirred at 5°C for 1 hour. Then the temperature of the obtained reaction mixture was elevated to 25°C, and the mixture was stirred for 2 hours. Water (3.74 L) was added to the reaction mixture to wash the mixture. The organic layer thus obtained was further washed with water. The obtained organic layer was concentrated under reduced pressure. Heptane was added to the obtained concentrate, and they were stirred at room temperature overnight. The crystals thus obtained were collected by the filtration to obtain (R)-1-[(4-methoxyphenyl)acetyl]pyrrolidine-2-carboxylic acid [2-(2-bromobenzyloxy)phenyl]amide (1.69 kg, 90 %).
¹H NMR (CDCl₃) δ 1.82-2.03(m,2H), 2.12(m,1H), 2.47(m,1H), 3.40-3.60(m,4H), 3.75(s,3H), 4.78(dd,J=8.0,1.7Hz,1H), 5.17(s,2H), 6.78(d-like,J=8.6Hz,2H), 6.87(dd,J=7.5,1.6Hz,1H), 6.93-7.03(m,2H), 7.10(d-like,J=8.6Hz,2H), 7,19(m, 1H), 7.28(dt,J=7.5,1.3Hz,1H), 7.55-7.62(m,2H), 8.35(dd,J=7.4,2.2Hz,1H), 9.27(br,1H).
¹³C NMR (CDCl₃) δ 25.0, 27.6, 40.7, 47.6, 55.2, 60.9, 70.3, 111.8, 114.0, 120.4, 121.5, 123.8, 126.1, 127.7, 129.4, 129.5, 130.0, 132.6, 135.9, 147.2, 158.5, 169.3, 171.5.
ESI MASS m/z (M⁺) 523.

### [Example 2]

### Preparation of (R)-[[2-(5,11-dihydro-dibenzo[b,e][1,4]oxazepine-5-carbonyl)pyrrolidin]-1-yl]-2-(4-methoxyphenyl)ethanone (5):

(R)-1-[(4-Methoxyphenyl)acetyl]pyrrolidine-2-carboxylic acid [2-(2-bromobenzyloxy)phenyl]amide (1.69 kg, 3.20 mol), cuprous bromide (23.0 g, 0.16 mol) and potassium carbonate (443 g, 3.20 mol) were added to pyridine (4.19 L), and the mixture was stirred under reflux in nitrogen atmosphere for 100 hours. After cooling to room temperature, toluene (8.4 L) was added to the reaction mixture. After washing the mixture with 6 M hydrochloric acid (9.17 L, 55.0 mol), the obtained organic layer was successively washed with 1 M hydrochloric acid, saturated aqueous sodium hydrogencarbonate solution and water. The organic layer was dried, the solvent was evaporated under reduced pressure, and the obtained residue was subjected to silica gel column chromatography and then eluted with a solvent mixture of ethyl acetate and hexane (3:2) to obtain (R)-[[2-(5,11-dihydro-dibenzo[b,e][1,4]oxazepine-5-carbonyl) pyrrolidin]-1-yl]-2-(4-methoxyphenyl)ethanone (1.35 kg, 95 %) in the form of a colorless oil.
¹H NMR (CDCl₃) δ 1.7-2.4(m,4H), 3.4-3.9(m,4H), 3.77(s,3H), 4.7-4.8(m, 2H), 5.73(m,1H), 6.4(m,1H), 6.7-7.6(m,11H). ESI MASS m/z 443 (MH⁺), 465 (M+Na⁺).

### [Example 3]

### Preparation of (R)-(+)-5,11-dihydro-5-[1-(4-methoxyphenethyl)-2-pyrrolidinylmethyl]dibenzo[b,e][1,4]oxazepine (6) hydrochloride:

Sodium borohydride (400 g, 10.57 mol) was suspended in tetrahydrofuran (10.77 L), and the obtained suspension was cooled to 5°C. A solution of (R)-[[2-(5,11-dihydro-dibenzo[b,e][1,4]oxazepine-5-carbonyl)pyrrolidin]-1-yl]-2-(4-methoxyphenyl)ethanone (1.35 kg, 3.04 mol) in tetrahydrofuran (2.69 L) was added to the suspension in nitrogen atmosphere. Boron trifluoride / tetrahydrofuran complex (1.97 kg, 14.10 mol) was added dropwise to the obtained mixture while the temperature was kept at 10°C or lower. After the completion of the addition, the reaction mixture was stirred at 5°C for 1 hour and then the temperature was elevated to 40°C, and the mixture was stirred for 14 hours. After cooling to 5°C, 1.5 M aqueous sodium hydroxide solution (13.6 L) was added dropwise to the reaction mixture. After the completion of the addition, the reaction mixture was stirred at 60°C for 2 hours. The solution was cooled to room temperature. After the extraction with toluene (8.1 L), the obtained organic layer was concentrated to a volume of about 7.5 L under reduced pressure, and then washed with water 3 times. The temperature of the organic layer was elevated to 30°C, to which 4 M hydrogen chloride / ethyl acetate solution (0.941 L, 3.01 mol) was added dropwise, and the obtained mixture was stirred at 5°C overnight. The crystals thus formed were collected by the filtration and then recrystallized from 2-propanol to obtain (R)-(+)-5,11-dihydro-5-[1-(4-methoxyphenethyl)-2-pyrrolidinylmethyl]dibenzo[b,e][1,4]oxazepine hydrochloride (1.00 kg, 73 %) in the form of white crystals.

The spectrum of this compound coincided with that given in WO 97/33885.

### [Example 4]

### Preparation of crystals 1 of (R)-[[2-(5,11-dihydro-dibenzo [b,e][1,4]oxazepine-5-carbonyl)pyrrolidin]-1-yl]-2-(4-methoxyphenyl) ethanone (5):

(R)-1-[(4-methoxyphenyl)acetyl]pyrrolidine-2-carboxylic acid [2-(2-bromobenzyloxy)phenyl]amide (1.26 kg, 2.40 mol), cuprous bromide (17.3 g, 0.12 mol) and potassium carbonate (996 g, 7.20 mol) were added to 4-picoline (3.14 L), and the mixture was stirred in nitrogen atmosphere at 130°C for 19 hours. After cooling to room temperature, toluene (6.28 L) was added to the reaction mixture and then 6 M hydrochloric acid (7.38 L, 44.3 mol) was added thereto to wash it. The obtained organic layer was successively washed with 1 M hydrochloric acid, saturated aqueous sodium hydrogencarbonate solution and water. The organic layer was concentrated at 25°C under reduced pressure to adjust the concentration of (R)-[[2-(5,11-dihydro-dibenzo [b,e][1,4] oxazepine-5-carbonyl) pyrrolidin]-1-yl]-2-(4-methoxyphenyl)ethanone to 50 wt. %. The solution was left to stand at room temperature overnight. The yellowish white crystals of (R)-[[2-(5,11-dihydro-dibenzo[b,e][1,4]oxazepine-5-carbonyl)pyrrolidin]-1-yl]-2-(4-methoxyphenyl)ethanone (733 g, 69 %)were obtained by the filtration. The area purity of (R)-[[2-(5,11-dihydro-dibenzo[b,e][1,4]oxazepine-5-carbonyl)pyrrolidin]-1-yl]-2-(4-methoxyphenyl)ethanone in HPLC was improved from 83.1 % to 99.1 % by the crystallization.
¹H NMR (CDCl₃) δ 1.7-2.4(m,4H), 3.4-3.9(m,4H), 3.77(s,3H), 4.7-4.8(m,2H), 5.73(m,1H), 6.4(m,1H), 6.7-7.6(m,11H).
ESI MASS m/z 443 (MH+), 465 (M+Na⁺).
Melting point: 132-134°C
Powder X ray crystal analysis: 2 θ = 7.9° 9.0° 14.4° 23.8°

### [Example 5]

### Preparation of crystals 2 of (R)-[[2-(5,11-dihydro-dibenzo [b,e][1,4]oxazepine-5-carbonyl)pyrrolidin]-1-yl]-2-(4-methoxyphenyl) ethanone (5):

(R)-1-[(4-methoxyphenyl)acetyl]pyrrolidine-2-carboxylic acid [2-(2-bromobenzyloxy)phenyl]amide (1.07 kg, 2.00 mol), cuprous bromide (14.4 g, 0.1 mol) and potassium carbonate (829 g, 6.00 mol) were added to 4-picoline (2.62 L), and the mixture was stirred in nitrogen atmosphere at 130°C for 20 hours. After cooling to room temperature, toluene (5.23 L) was added to the reaction mixture and then 6 M hydrochloric acid (6.15 L, 36.9 mol) was added thereto to wash it. The obtained organic layer was successively washed with 1 M hydrochloric acid, saturated aqueous sodium hydrogencarbonate solution and water. The organic layer was concentrated at 50°C under reduced pressure to adjust the concentration of (R)-[[2-(5,11-dihydro-dibenzo [b,e][1,4] oxazepine-5-carbonyl) pyrrolidin]-1-yl]-2-(4-methoxyphenyl)ethanone to 20 wt. %. The solution was stirred overnight and then cooled to 5°C. The yellowish white crystals of (R)-[[2-(5,11-dihydro-dibenzo[b,e][1,4]oxazepine-5-carbonyl)pyrrolidin]-1-yl]-2-(4-methoxyphenyl)ethanone (733 g, 69 %) thus formed were obtained by the filtration. The area purity of (R)-[[2-(5,11-dihydro-dibenzo[b,e][1,4]oxazepine-5-carbonyl)pyrrolidin]-1-yl]-2-(4-methoxyphenyl)ethanone in HPLC was improved from 89.4 % to 97.8 % by the crystallization.
¹H NMR (CDCl₃) δ 1.7-2.4(m,4H), 3.4-3.9(m,4H), 3.77(s,3H), 4.7-4.8(m,2H), 5.73(m,1H), 6.4(m,1H), 6.7-7.6(m,11H).
ESI MASS m/z 443 (MH⁺), 465 (M+Na⁺).
Melting point: 148-150°C
Powder X ray crystal analysis: 2 θ = 12.50 18.5° 19.3° 21.1° 21.4°

### [Example 6]

### Transition of crystals 1 of (R)-[[2-(5,11-dihydro-dibenzo[b,e][1,4] oxazepine-5-carbonyl)pyrrolidin]-1-yl]-2-(4-methoxyphenyl)ethanone (5) to crystals 2 thereof:

Crystals 1 of (R)-[[2-(5,11-dihydro-dibenzo[b,e][1,4]oxazepine-5-carbonyl)pyrrolidin]-1-yl]-2-(4-methoxyphenyl)ethanone (1.34 g) obtained in Example 4 was suspended in toluene (6 mL), and the obtained suspension was stirred at 10 °C for 47 hours. The suspension was filtered to obtain yellowish white crystals 2 of (R)-[[2-(5,11-dihydro-dibenzo[b,e][1,4]oxazepine-5-carbonyl)pyrrolidin]-1-yl]-2-(4-methoxyphenyl)ethanone (1.04 g, 78 %).

Thus, 5-substituted-5,11-dihydro-dibenzo[b,e][1,4]oxazepine derivatives, in particular, 5,11-dihydro-5-[1-(4-methoxyphenethyl)-2-pyrrolidinylmethyl]dibenzo[b,e][1,4]oxazepine, could be obtained in a high yield. The industrially useful process could be provided.

## Claims

1. A process for producing 5-substituted-5,11-dihydro-dibenzo [b,e][1,4]oxazepine derivatives represented by the following formula (3) and stereoisomers thereof: 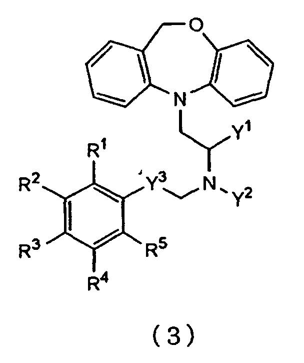 wherein Y¹ represents hydrogen atom, Y² represents hydrogen atom or a lower alkyl group, or Y¹ and Y² together represent CH₂-CH₂-CH₂ or CH₂-CH₂-CH₂-CH₂, Y³ represents CH₂ or CH₂-CH₂, and R¹ to R⁵ may be the same or different from one another and they each represent hydrogen atom, a halogen atom, a lower alkyl group, hydroxyl group, a lower alkoxyl group, amino group or a lower alkylamino group, or R¹ and R², R² and R³, R³ and R⁴ or R⁴ and R⁵ together form -OCH₂O-
which comprises the steps of intramolecular-arylating a [2-(2-bromobenzyloxy)phenyl]amide derivative of the following formula (1): 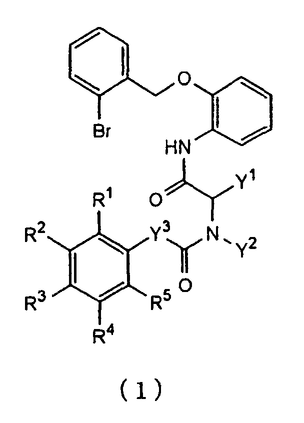 wherein Y¹, Y² and Y³ and R¹ to R⁵ are as defined above
to form a 5,11-dihydro-dibenzo[b,e][1,4]oxazepine derivative of the following formula (2): 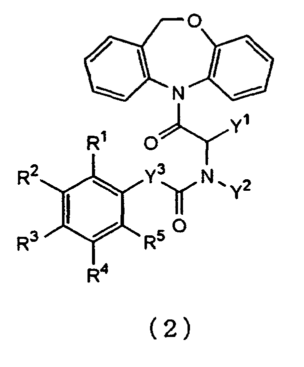 wherein Y¹, Y² and Y³ and R¹ to R⁵ are as defined above
and reducing this compound.

2. The process of claim 1, wherein the [2-(2-bromo benzyloxy)phenyl]amide derivative of the formula (1) is (R)-1-[(4-methoxyphenyl)acetyl]pyrrolidine-2-carboxylic acid [2-(2-bromo benzyloxy)phenyl]amide of the following formula (4), 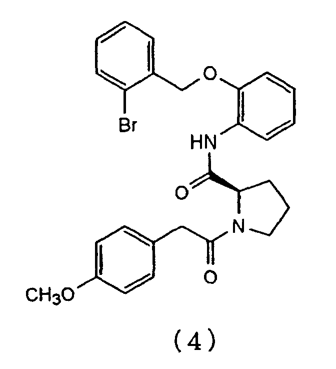 the 5,11-dihydro-dibenzo[b,e][1,4]oxazepine derivative represented by the formula (2) is (R)-[[2-(5,11-dihydro-dibenzo[b,e][1,4]oxazepine-5-carbonyl)pyrrolidin]-1-yl]-2-(4-methoxyphenyl)ethanone of the following formula (5), 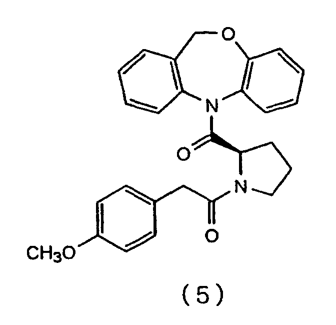 and the 5-substituted-5,11-dihydro-dibenzo[b,e][1,4]oxazepine derivative of the formula (3) or the stereoisomer thereof is (R)-(+)-5,11-dihydro-5-[1-(4-methoxyphenethyl)-2-pyrrolidinylmethyl]dibenzo[b,e][1,4]-oxazepine of the following formula (6):

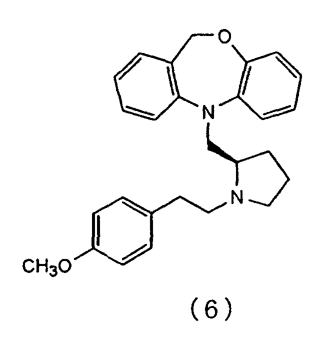

3. The process of claim 2 which comprises the steps of crystallizing (R)-[[2-(5,11-dihydro-dibenzo[b,e][1,4]oxazepine-5-carbonyl)pyrrolidin]-1-yl]-2-(4-methoxyphenyl)ethanone of the above formula (5) obtained by the intramolecular arylation, isolating and then reducing the resulting crystals.

4. A process for producing (R)-(+)-5,11-dihydro-5-[1-(4-methoxyphenethyl)-2-pyrrolidinylmethyl]dibenzo[b,e][1,4]oxazepine of the following formula (6), which comprises the steps of crystallizing (R)-[[2-(5,11-dihydro-dibenzo[b,e][1,4]oxazepine-5-carbonyl)pyrrolidin]-1-yl]-2-(4-methoxyphenyl)ethanone of the following formula (5), isolating and then reducing the resulting crystals:

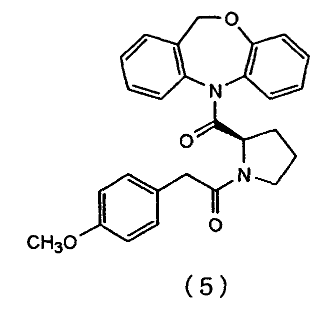

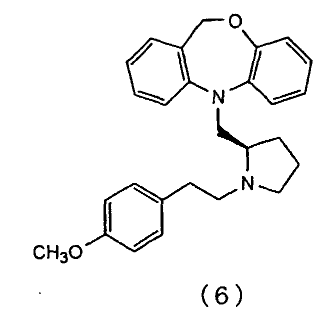

5. (R)-1-[(4-Methoxyphenyl)acetyl]pyrrolidine-2-carboxylic acid [2-(2-bromobenzyloxy)phenyl]amide of the following formula (4):

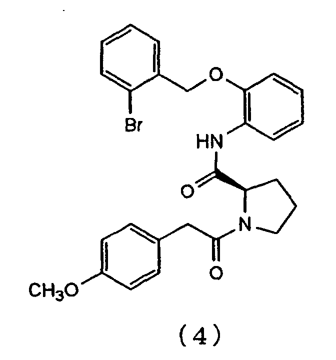

6. (R)-[[2-(5,11-Dihydro-dibenzo[b,e][1,4]oxazepine-5-carbonyl) pyrrolidin]-1-yl]-2-(4-methoxyphenyl)ethanone of the following formula (5):

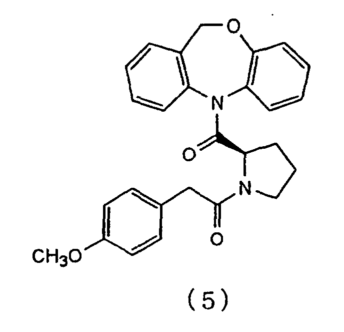

7. Crystals of (R)-[[2-(5,11-dihydro-dibenzo[b,e][1,4]oxazepine-5-carbonyl)pyrrolidin]-1-yl]-2-(4-methoxyphenyl)ethanone.

8. Crystals of claim 7, which satisfy at least one of the following conditions a and b:
a: melting point: 132 to 134°C, and
b: powder X ray crystal analysis: 2 θ = 7.9° 9.0° 14.4° 23.8°

9. Crystals of claim 7, which satisfy at least one of the following conditions a and b:
a: melting point: 148 to 150°C, and
b: powder X ray crystal analysis: 2 θ = 12.5° 18.5° 19.3° 21.1° 21.4°
